# EUROPEAN PATENT SPECIFICATION

(11) **EP 1 549 286 B1**
(45) Date of publication and mention of the grant of the patent: **04.07.2012**
(21) Application number: 03808100.6
(22) Date of filing: 30.09.2003
(51) Int. Cl.: A61K 8/81, A61Q 19/00

(54) **COSMETIC COMPOSITIONS WITH REDUCED TACK**
KOSMETISCHE ZUSAMMENSETZUNGEN MIT VERRINGERTER KLEBRIGKEIT
COMPOSITIONS COSMETIQUES A PEGOSITE REDUITE

(30) Priority: 09.10.2002 US 417101 P
(43) Date of publication of application: 06.07.2005
(73) Proprietor: THE PROCTER & GAMBLE COMPANY, Cincinnati, OH 45202 (US)
(72) Inventor: DECKNER, George, Endel, Cincinnati, OH 45249 (US); LEBLANC, Micahel, Jude, Cincinnati, OH 45219 (US); STEPHENS, Alison, Fiona, Cookham, Berkshire SL6 9LA (GB); CROOK, Teresa, Barbara, Camberley, Surrey GU17 9LY (GB)
(74) Representative: Simpson, Kirsty Mairi
(86) International application number: PCT/US2003/030826
(87) International publication number: WO 2004/032894

(56) References cited:
- WO-A-01/51013
- WO-A-01/76552

## Description

### TECHNICAL FIELD

Cosmetic compositions in the form of oil-in-water emulsions that comprise high levels of water-soluble skin benefit agents in combination with cross-linked co-polymers are provided. The cosmetic compositions have increased skin moisturization with reduced tack.

### BACKGROUND

A wide variety of cosmetic compositions have been used to treat dry skin. These compositions typically contain lipophilic moisturizing agents that moisturise the skin via occlusion that inhibits or reduces water loss. These compositions can additionally comprise other water-soluble skin benefit agents such as vitamins and humectants. Addition of these ingredients can increase the moisturisation of the skin, or create other effects on the skin such as desquamation.

However, it can be difficult to produce cosmetic compositions comprising these ingredients that do not leave a tacky residue on the skin. These compositions, when applied to the skin, lose water via evaporation and skin absorption. The loss of water leads to an increase in the effective concentration of moisturizing agents, water-soluble skin benefit agents and thickeners, which in turn leads to an increase in the viscosity of the dried film and the perceived tackiness of the residue. This problem may be exacerbated by increasing the relative amount of water-soluble skin benefit agents within the composition. Thus, although cosmetic compositions having higher concentrations of water-soluble skin benefit agents are generally desired due to their increased skin benefit, such compositions are typically too tacky to be acceptable to the consumer.

Previously, the increased tack caused by high levels of water-soluble skin benefit agents has been mitigated using a variety of methods. For example, US 4,389,418 discloses the use of a quaternary ammonium compound as the sole emulsifier in an oil-in-water emulsion to reduce tack. US 5,804,205 discloses the use of quaternary ammonium compounds in combination with hydrophobic polymeric microspheres having an average particle size of less than 50 µm to reduce tack. Also, lipophilic copolymer thickeners having lipophilic and hydrophilic monomer units have been described as being useful in solving the problem of tack in skin compositions such as in WO 02/058665 A1 and US 6,433,061 B1. Whilst these compositions reduce tack, some may have poor akin feel characteristics such as drag when topically applied. Furthermore, some of these co-polymer compositions may be susceptible to salt-shrinkage due to the salt present on the skin. This often results in the compositions "breaking" on the skin and feeling watery and/or greasy.

Therefore, it is desirable to provide cosmetic compositions comprising high levels of water-soluble skin benefit agents that have good moisturization characteristics when topically applied to the skin. Furthermore, it is desirable to provide cosmetic compositions comprising high levels of water-soluble skin benefit agents with reduced tack when topically applied to the skin. Finally, it is desirable to provide cosmetic compositions comprising high levels of water-soluble skin benefit agents that have improved consumer aesthetics and reduced drag.

### SUMMARY

A cosmetic composition is provided in the form of an oil-in-water emulsion, wherein the total level of oil phase components in the composition is between 5 and 15% by weight of the composition and wherein the continuous aqueous phase comprises from 0.1 to 2% cross-linked co-polymer by weight of the composition, the cross-linked co-polymer comprising;
i) from 35% to 94.5% water-soluble anionic acrylic monomers by weight of the cross-linked co-polymer;
ii) from 5% to 65% water-soluble non-ionic acrylate monomers by weight of the cross-linked co-polymer; and
iii) from 0.005% to 0.5% bifunctional monomeric crosslinking agent by weight of the cross linked co-polymer;
the composition further comprising greater than 10% of a water soluble skin benefit agent by weight of the composition. The compositions of the present invention exhibit good stability, excellent skin feel characteristics and reduced tack or stickiness when topically applied to the skin.

Herein, "water soluble" includes materials that are soluble in water at 25°C at a level of at least 3.3% by weight, as defined in the European pharmacopoeia monographs (ISSN 1013-5294 Technical Guide for the elaboration of monographs, 3rd Ed, pgs 86-87).

These and other features, aspects, and advantages of the present invention will become evident to those skilled in the art from a reading of the present disclosure.

### DETAILED DESCRIPTION

All weights, measurements and concentrations herein are measured at 25°C on the composition in its entirety, unless otherwise specified.

Unless otherwise indicated, all percentages of compositions referred to herein are weight percentages and all ratios are weight ratios.

Unless otherwise indicated, all molecular weights are weight average molecular weights.

Unless otherwise indicated, the content of all literature sources referred to within this text are incorporated herein in full by reference.

Except where specific examples of actual measured values are presented, numerical values referred to herein should be considered to be qualified by the word "about"

The cosmetic compositions herein comprise a continuous aqueous phase comprising a cross-linked co-polymer. The cross-linked co-polymer comprises from 35% to 95%, preferably from 50% to 70%, water-soluble anionic acrylic monomers, from 5% to 65% water-soluble non-ionic acrylate monomers, and from 0.005% to 0.5% bifunctional monomeric cross-linking agent by weight of the cross-linked co-polymer.

Suitable water-soluble anionic acrylic-based monomers include acrylic acid, methacrylic acid or mixtures thereof.

Suitable water-soluble non-ionic acrylate-based monomers include acrylamide, methacrylamide, N-vinyl pyrolidone, water-soluble hydroxy-substituted acrylic or methacrylic esters or mixtures thereof.

Suitable bifunctional monomeric cross-linking agents include di, tri and tetraethylenically unsaturated materials such as methylene bis acrylamide, divinylpyrroline and allyl (meth) acrylate or mixtures thereof.

Commercial examples of co-polymer compositions suitable for use herein include the co-polymer compositions commercially available from BASF Corp. under the tradename Luvigel® EM and the co-polymer compositions available from CIBA Speciality Chemicals, Macclesfield, UK, under the tradename Salcare SC91.

The cross-linked co-polymer comprises from 0.1% to 2% by weight of the composition. Preferably, the cross-linked co-polymer comprises from 0.2% to 1.5%, more preferably 0.3% to 1.0% by weight of the composition.

Surprisingly, it has been found that these cross-linked co-polymers significantly reduce the tack associated with compositions comprising a high proportion of water-soluble skin benefit agent. Without wishing to be bound by theory, it is believed that these co-polymers reduce tack by forming a film over the external surface of the composition when applied to the skin. It is believed that a film is formed by the co-polymer composition that is imperceptible to consumers and traps the water and water-soluble skin benefit agent contained therein between the skin and the film, preventing the composition from appearing tacky to the user. The presence of water-soluble non-ionic acrylate monomers in the system is observed to be important in achieving this effect, although the precise mechanism is not understood. It is believed that these polymers enable film formation by providing a plasticising effect when suspended in water. This film-formation also allows the compositions herein to have reduced drag and enhanced consumer aesthetics when topically applied.

The composition of the present invention comprises greater than 10%, preferably greater than 10% up to 40%, more preferably from 12% to 30%, and more preferably still from 17% to 27% by weight of a water-soluble skin benefit agent. Water-soluble skin benefit agents useful herein include vitamin B₃ compounds, humectants, amino acids, vitamin C compounds, panthenol and derivatives, or mixtures thereof. Incorporation of greater than 10% water-soluble skin benefit agent in the present compositions enhances the skin benefit properties by providing reduced water loss and/or providing a desquamatory, keratolytic and rejuvenating effect when topically applied.

One class of water-soluble skin benefit agent according to the present invention includes vitamin B₃ compounds. As used herein, "vitamin B₃ compound" includes compounds having the formula: 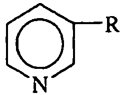 wherein R is - CONH₂ (i.e., niacinamide), - COOH (i.e., nicotinic acid) or - CH₂OH (i.e., nicotinyl alcohol); derivatives thereof; and salts of any of the foregoing. Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of C₁-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye). Non-vasodilating esters of nicotinic acid include tocopherol nicotinate and inositol hexanicotinate; tocopherol nicotinate is preferred.

Another class of water-soluble skin benefit agent according to the present invention comprises humectants. Suitable humectants useful herein include polyhydric alcohols, sodium 2-pyrrolidone-5-carboxylate (NaPCA), guanidine; glycolic acid and glycolate salts (e.g. ammonium and quaternary alkyl ammonium); lactic acid and lactate salts (e.g. ammonium and quaternary alkyl ammonium); other alpha hydroxy acids such as malic acid, aloe vera in any of its variety of forms (e.g., aloe vera gel); hyaluronic acid, precursors and derivatives thereof (e.g., glucosamine and salt derivatives such as sodium hyaluronate); lactamide monoethanolamine; acetamide monoethanolamine; urea; and mixtures thereof.

Preferred humectants, from the viewpoint of boosting moisturisation, are the polyhydric alcohols. Suitable polyhydric alcohols for use herein include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (e.g., 1,3-butylene glycol), hexane triol (e.g., 1,2,6-hexanetriol), trimethylol propane, neopentyl glycol, glycerine, ethoxylated glycerine and propoxylated glycerine. Preferred polyhydric alcohols of the present invention are polyhydric alcohols with 3 to 9 carbon atoms in the molecule. Suitable polyhydric alcohols include glycerine, butylene glycol, propylene glycol, dipropylene glycol, polyethylene glycol and derivatives thereof, hexane triol, ethoxylated glycerine and propoxylated glycerine, or mixtures thereof. More preferred for use in the present invention is glycerin.

A further class of water-soluble skin benefit agent according to the present invention comprises panthenol or its derivatives. The panthenol and its derivatives include D-panthenol ([R]-2,4-dihydroxy-N-[3-hydroxypropyl)]-3,3-dimethylbutamide), DL-panthenol, calcium pantothenate, royal jelly, panthetine, pantotheine, panthenyl ethyl ether, pangamic acid, pyridoxin, pantoyl lactose and Vitamin B complex.

A further class of water-soluble skin benefit agent according to the present invention comprises amino acids and their derivatives. Suitable amino acids for use herein include both D- and L- isomers of naturally occurring amino acids. Suitable examples include L-isomers of serine, alanine, proline and hydroxyproline.

An additional class of water-soluble skin benefit agent according to the present invention comprises a vitamin C compound. Vitamin C compounds include water-soluble ascorbic acid salts and esters thereof. Vitamin C compounds are particularly useful as skin lightening agents. Suitable examples include magnesium ascorbyl phosphate and the sodium salt of the monophosphate ester of ascorbic acid, commercially available from Roche Vitamins Europe Ltd as Stay-C 50.

The compositions of the invention are preferably formulated so as to have a product viscosity of at least about 4,000 mPa.s and preferably in the range from about 4,000 to about 300,000 mPa.s, more preferably from about 8,000 to about 250,000 mPa.s and especially from about 10,000 to about 200,000 mPa.s and even more especially from about 10,000 to about 150,000 mPa.s (25°C, neat, Brookfield RVT, T-C Spindle at 5 rpms and Heliopath Stand).

The cosmetic compositions herein are in the form of an emulsion of one or more oil phases in an aqueous continuous phase, each oil phase comprising a single oily component or a mixture of oily components in miscible or homogeneous form. Different oil phases contain different materials, or different combinations of materials, from each other. The total level of oil phase components in the compositions of the invention is typically from from 5% to 15%.

In preferred embodiments, the oil phase preferably comprises oily components such as a natural or synthetic oils selected from mineral, vegetable, and animal oils, fats and waxes, fatty acid esters, fatty alcohols, fatty acids and mixtures thereof. Preferred for use herein are for example, saturated and unsaturated fatty alcohols such as behenyl alcohol, cetyl alcohol and stearyl alcohol and hydrocarbons such as mineral oils or petrolatum.

The present compositions may comprise at least one silicone phase. The silicone phase can comprise one or more silicone components such as silicone fluids, gums, and mixtures thereof, The, or each, silicone phase generally comprises from about 0.1% to about 20%, preferably from about 0.2% to about 10%, more preferably from about 0.3% to about 5%, of the composition.

Silicone components can be fluids, including straight chain, branched and cyclic silicones. Suitable silicone fluids useful herein include silicones inclusive of polyalkyl siloxane fluids, polyaryl siloxane fluids, cyclic and linear polyalkylsiloxanes, polyalkoxylated silicones, amino and quaternary ammonium modified silicones, polyalkylaryl siloxanes or a polyether siloxane copolymer and mixtures thereof. The silicone fluids can be volatile or non-volatile. Silicone fluids generally have a weight average molecular weight of less than about 200,000. Suitable silicone fluids have a molecular weight of about 100,000 or less, preferably about 50,000 or less, more preferably about 10,000 or less. Preferably the silicone fluid is selected from silicone fluids having a weight average molecular weight in the range from about 100 to about 50,000 and preferably from about 200 to about 40,000. Typically, silicone fluids have a viscosity ranging from about 0.65 to about 600,000 mm².s⁻¹, preferably from about 0.65 to about 10,000 mm².s⁻¹ at 25°C. The viscosity can be measured by means of a glass capillary viscometer as set forth in Dow Coming Corporate Test Method CTM0004, July 29, 1970. Suitable polydimethyl siloxanes that can be used herein include those available, for example, from the General Electric Company as the SF and Viscasil (RTM) series and from Dow Coming as the Dow Coming 200 series. Also useful are essentially non-volatile polyalkylarylsiloxanes, for example, polymethylphenylsiloxanes, having viscosities of about 0.65 to 30,000 mm².s⁻¹ at 25°C. These siloxanes are available, for example, from the General Electric Company as SF 1075 methyl phenyl fluid or from Dow Coming as 556 Cosmetic Grade Fluid. Cyclic polydimethylsiloxanes suitable for use herein are those having a ring structure incorporating from about 3 to about 7 (CH₃)₂SiO moieties.

Another class of silicone components suitable for use in a silicone oil phase herein includes polyether siloxane copolymers. Examples of polyether siloxan copolymers include polydiorganosiloxane-polyoxyalkylene copolymers containing at least one polydiorganosiloxane segment and at least one polyoxyalkylene segment. Suitable polydiorganosiloxane-polyalkylene copolymers are available commercially under the tradenames Belsil (RTM) from Wacker-Chemie GmbH, Geschäftsbereich S, Postfach D-8000 Munich 22 and Abil (RTM) from Th. Goldschmidt Ltd., Tego House, Victoria Road, Ruislip, Middlesex, HA4 0YL, for example Belsil (RTM) 6031 and Abil (RTM) B88183. A particularly preferred copolymer fluid blend for use herein includes Dow Coming DC5225C which has the CTFA designation Dimethicone/Dimethicone copolyol.

In preferred embodiments, the silicone fluid is selected from dimethicone, decamethylcyclopentasiloxane, octamethylcyclotetrasiloxane, phenyl methicone, and mixtures thereof.

The silicone components can also comprise silicone gums. The term "silicone gum" herein includes high molecular weight silicones having a weight average molecular weight in excess of about 200,000 and preferably from about 200,000 to about 4,000,000. Included are non-volatile polyalkyl and polyaryl siloxane gums. In preferred embodiments, a silicone oil phase comprises a silicone gum or a mixture of silicones including the silicone gum. Typically, silicone gums have a viscosity at 25°C in excess of about 1,000,000 mm²s⁻¹. The silicone gums include dimethicones as described in Noll, Walter, Chemistry and Technology of Silicones, New York: Academic Press 1968. Also describing silicone gums are General Electric Silicone Rubber Product Data Sheets SE 30, SE 33, SE 54 and SE 76. Specific examples of silicone gums include polydimethylsiloxane, (polydimethylsiloxane)(methylvinylsiloxane) copolymer, poly(dimethylsiloxane)(diphenyl)(methylvinylsiloxane) copolymer and mixtures thereof. Preferred silicone gums for use herein are silicone gums having a molecular weight of from about 200,000 to about 4,000,000 selected from dimethiconol, and dimethicone and mixtures thereof.

Preferably the silicone phase herein comprises a silicone gum incorporated into the composition as part of a silicone gum-fluid blend. When the silicone gum is incorporated as part of a silicone gum-fluid blend, the silicone gum preferably constitutes from about 5% to about 40%, especially from about 10% to 20% by weight of the silicone gum-fluid blend. Suitable silicone gum-fluid blends herein are mixtures consisting essentially of:
(i) a silicone having a molecular weight of from about 200,000 to about 4,000,000 selected from dimethiconol, fluorosilicone and dimethicone and mixtures thereof; and
(ii) a carrier which is a silicone fluid, the carrier having a viscosity from about 0.65 mm².s⁻¹ to about 100 mm².s⁻¹,
wherein the ratio of i) to ii) is from about 10:90 to about 20:80 and wherein said silicone gum-based component has a final viscosity of from about 100 mm².s⁻¹ to about 100,000 mm².s⁻¹, preferably from 500 mm².s⁻¹ to about 10,000 mm².s⁻¹.
An especially preferred silicone-gum fluid blend based component for use in the compositions herein is a dimethiconol gum having a molecular weight of from about 200,000 to about 4,000,000 along with a silicone fluid carrier with a viscosity of about 0.65 to 100 mm².s⁻¹. An example of this silicone component is Dow Coming Q2-1503 (85% 5 mm².s⁻¹ Dimethicone Fluid/15% Dimethiconol) and Dow Coming Q2-1501 available from Dow Coming.

The compositions of the present invention can comprise emollient materials including branched chain hydrocarbons having an weight average molecular weight of from about 100 to about 15,000, preferably from about 100 to 1000; compounds of formula I: 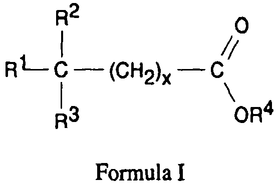 wherein R¹ is selected from H or CH₃, R², R³ and R⁴ are independently selected from C₁-C₂₀ straight chain or branched chain alkyl, and x is an integer of from 1-20; and compounds having the formula (II): 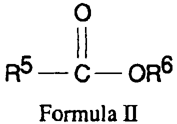 wherein R⁵ is selected from optionally hydroxy or C₁-C₄ alkyl substituted benzyl and R₆ is selected from C₁-C₂₀ branched or straight chain alkyl; and mixtures thereof.

Suitable branched chain hydrocarbons for use herein include isododecane, isohexadecane, isoeicosane, isooctahexacontane, isohexapentacontahectane, isopentacontaoctactane, and mixture thereof. Suitable for use herein are branched chain aliphatic hydrocarbons sold under the trade name Permethyl (RTM) and commercially available from Presperse Inc., P.O. Box 735, South Plainfield, N.J. 07080, U.S.A. Suitable ester emollient materials of Formula I above include, but are not limited to, methyl isostearate, isopropyl isostearate, isostearyl neopentanoate, isononyl isononanoate, isodecyl octanoate, isodecyl isononanoate, tridecyl isononanoate, myristyl octanoate, octyl pelargonate, octyl isononanoate, myristyl myristate, myristyl neopentanoate, myristyl octanoate, myristyl propionate, isopropyl myristate and mixtures thereof. Suitable ester emollient materials of Formula (II) include but are not limited to C12-15 alkyl benzoates.

Preferred emollients for use herein are isohexadecane, isooctacontane, isononyl isononanoate, isodecyl octanoate, isodecyl isononanoate, tridecyl isononanoate, myristyl octanoate, octyl isononanoate, myristyl myristate, methyl isostearate, isopropyl isostearate, C12-15 alkyl benzoates and mixtures thereof. Particularly preferred emollients for use herein are isohexadecane, isononyl isononanoate, methyl isostearate, isopropyl isostearate, or mixtures thereof. A further emollient suitable for use in the composition of the present invention is petrolatum.

The emollient material is preferably present in the compositions at a level of from about 0.1% to about 10%.

The present compositions herein may comprise an emulsifier and/or surfactant, generally to help disperse and suspend the discontinuous phase within the continuous phase. A surfactant may also be useful if the product is intended for skin cleansing. For convenience hereinafter emulsifiers will be referred to under the term 'surfactants', thus 'surfactant(s)' will be used to refer to surface active agents whether used as emulsifiers or for other surfactant purposes such as skin cleansing. Known or conventional surfactants can be used in the composition, provided that the selected agent is chemically and physically compatible with essential components of the composition, and provides the desired characteristics. Suitable surfactants include silicone materials, non-silicone materials, and mixtures thereof.

The compositions of the present invention preferably comprise from about 0.05% to about 15% of a surfactant or mixture of surfactants. The exact surfactant or surfactant mixture chosen will depend upon the pH of the composition and the other components present.

Preferred surfactants are nonionic. Among the nonionic surfactants that are useful herein are those that can be broadly defined as condensation products of long chain alcohols, e.g. C₈₋₃₀ alcohols, with sugar or starch polymers, i.e., glycosides. These compounds can be represented by the formula (S)ₙ-O-R wherein S is a sugar moiety such as glucose, fructose, mannose, and galactose; n is an integer of from about 1 to about 1000, and R is a C₈₋₃₀ alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples include a mixture of cetearyl glucosides and cetearyl alcohols such as those commercially available as Montanov 68™ from Seppic and Emulgade PL68/50™ available from Henkel.

Other useful nonionic surfactants include the condensation products of alkylene oxides with fatty acids (i.e. alkylene oxide esters of fatty acids). These materials have the general formula RCO(X)ₙOH wherein R is a C₁₀₋₃₀ alkyl group, X is -OCH₂CH₂- (i.e. derived from ethylene glycol or oxide) or -OCH₂CHCH₃- (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula RCO(X)ₙOOCR wherein R is a C₁₀₋₃₀ alkyl group, X is -OCH₂CH₂-(i.e. derived from ethylene glycol or oxide) or -OCH₂CHCH₃-(i.e. derived from propylene glycol or oxide), and n is an integer from about 6 to about 100. Other nonionic surfactants are the condensation products of alkylene oxides with fatty alcohols (i.e. alkylene oxide ethers of fatty alcohols). These materials have the general formula R(X)ₙOR' wherein R is a C₁₀₋₃₀ alkyl group, X is -OCH₂CH₂-(i.e. derived from ethylene glycol or oxide) or -OCH₂CHCH₃- (i.e. derived from propylene glycol or oxide), and n is an integer from about 6 to about 100 and R' is H or a C10-30 alkyl group. Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. These materials have the general formula RCO(X)ₙOR' wherein R and R' are C₁₀₋₃₀ alkyl groups, X is -OCH₂CH₂ (i.e. derived from ethylene glycol or oxide) or - OCH₂CHCH₃- (derived from propylene glycol or oxide), and n is an integer from about 6 to about 100, examples of which include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Still other useful nonionic surfactants include polyhydroxy fatty acid amide surfactants, which are described in more detail in WO98/04241.

Preferred among the nonionic surfactants are those selected from the group consisting of steareth-2, steareth-21, ceteareth-20, ceteareth-12, sucrose cocoate, steareth-100, PEG-100 stearate, and mixtures thereof.

Other nonionic surfactants suitable for use herein include sugar esters and polyesters, alkoxylated sugar esters and polyesters, C₁-C₃₀ fatty acid esters of C₁-C₃₀ fatty alcohols, alkoxylated derivatives of C₁-C₃₀ fatty acid esters of C₁-C₃₀ fatty alcohols, alkoxylated ethers of C1-C30 fatty alcohols, polyglyceryl esters of C₁-C₃₀ fatty acids, C₁-C₃₀ esters of polyols, C₁-C₃₀ ethers of polyols, alkyl phosphates, polyoxyalkylene fatty ether phosphates, fatty acid amides, acyl lactylates, and mixtures thereof. Examples of these non-silicon-containing surfactants include: polysorbate 20, polyethylene glycol 5 soya sterol, steareth-20, ceteareth-20, PPG-2 methyl glucose ether distearate, ceteth-10, polysorbate 80, polysorbate 60, glyceryl stearate, sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof.

Another emulsifier useful herein are fatty acid ester blends based on a mixture of sorbitan or sorbitol fatty acid ester and sucrose fatty acid ester, the fatty acid in each instance being preferably C₈-C₂₄, more preferably C₁₀-C₂₀. The preferred fatty acid ester emulsifier is a blend of sorbitan or sorbitol C₁₆-C₂₀ fatty acid ester with sucrose C₁₀-C₁₆ fatty acid ester, especially sorbitan stearate and sucrose cocoate. This is commercially available from ICI under the trade name Arlatone 2121.

Anionic surfactants are also useful in the present compositions. See, e.g., U.S. Patent No. 3,929,678, to Laughlin et al., issued December 30, 1975. Exemplary anionic surfactants include the alkoyl isethionates (e.g., C₁₂ - C₃₀), alkyl and alkyl ether sulfates and salts thereof, alkyl and alkyl ether phosphates and salts thereof, alkyl methyl taurates (e.g., C₁₂ - C₃₀), and soaps (e.g., alkali metal salts, e.g., sodium or potassium salts) of fatty acids.

Amphoteric and zwitterionic surfactants are also useful herein. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C₈ - C₁₈) and one contains an anionic water solubilising group, e.g., carboxy, sulfonate, sulfate, phosphate, or phosphonate. Examples are alkyl imino acetates, and iminodialkanoates and aminoalkanoates, imidazolinium and ammonium derivatives. Other suitable amphoteric and zwitterionic surfactants are those selected from the group consisting of betaines, sultaines, hydroxysultaines, alkyl sarcosinates (e.g., C₁₂ - C₃₀), and alkanoyl sarcosinates.

Emulsions of the present invention may include a silicone containing emulsifier or surfactant. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols, i.e., compounds that contain C₂-C₃₀ pendant side chains. Still other useful dimethicone copolyols include materials having various cationic, anionic, amphoteric, and zwitterionic pendant moieties.

The composition of the present invention comprises water. Preferably, water comprises from 30% to 85% by weight of the composition, more preferably 50% to 75% by weight of the composition.

The compositions of the present invention may optionally include particulate materials. Particulate materials suitable herein include materials that are insoluble in both water and oil with a median particle size of from 1 µm to 50 µm. Preferably the compositions of the present invention comprise particulate materials having a refractive index of from about 1.3 to about 1.7, the particulate materials being dispersed in the composition and having a median particle size of from about 2 to about 30 µm.. Suitable particulate materials are organic or organosilicone or inorganic. Preferred particles are free-flowing, solid, materials. By "solid" is meant that the particles are not hollow. The void at the centre of hollow particles can have an adverse effect on refractive index and therefore the visual effects of the particles on either skin or the composition. Suitable organic particulate materials include those made of polymethylsilsesquioxane, polyamide, polythene, polyacrylonitrile, polyacrylic acid, polymethacrylic acid, polystyrene, polytetrafluoroethylene (PTFE) and poly(vinylidene chloride). Copolymers derived from monomers of the aforementioned materials can also be used. Inorganic materials include silica and boron nitride. Representative commercially available examples of useful particulate materials herein are Tospearl^{®} 145, Orgasol 2002, Nylonpoly WL10, Dry Flo or mixtures thereof.

The compositions of the present invention can comprise from about 0.1% to about 5% by weight of particulate materials.

Another optional ingredient are neutralizing agents. Neutralizing agents suitable for use in neutralizing acidic groups containing hydrophilic gelling agents herein include sodium hydroxide, potassium hydroxide, ammonium hydroxide, monoethanolamine, diethanolamine, amino methyl propanol, tris-buffer and triethanolamine.

A further optional component may comprise sunscreening agents. Preferred among those sunscreens which are useful in the compositions of the invention are those selected from 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, Parsol MCX, Eusolex 6300, Octocrylene, Parsol 1789, and mixtures thereof.

Generally, the sunscreens can comprise from about 0.5% to about 20% of the compositions useful herein. Exact amounts will vary depending upon the sunscreen chosen and the desired Sun Protection Factor (SPF). SPF is a commonly used measure of photoprotection of a sunscreen against erythema. See Federal Register, Vol. 43, No. 166, pp. 38206-38269, August 25, 1978.

Other optional materials herein include pigments which, where water-insoluble, contribute to and are included in the total level of oil phase ingredients. Pigments suitable for use in the compositions of the present invention can be organic and/or inorganic. Also included within the term pigment are materials having a low colour or lustre such as matte finishing agents, and also light scattering agents.

Further optional ingredients include oil-soluble actives. Suitable oil soluble actives for use herein include vitamin E and its derivatives, salicylic acid and other beta-hydroxy acids, sun protection factors known to those in the art, perfumes and occlusion materials, and mixtures thereof.

### EXAMPLES I - V

| **INGREDIENTS** | **I** | **II** | **III** | **IV** | **V** |
|---|---|---|---|---|---|
| | **% w/w** | **% w/w** | **% w/w** | **% w/w** | **% w/w** |
| DEIONISED WATER | QS | QS | QS | QS | QS |
| DISODIUM EDTA | 0.1 | 0.1 | 0.1 | 0.1 | 0.1 |
| GLYCERIN | 10.0 | 15.0 | 20.0 | 25.0 | 15.0 |
| MACINAMIDE | 3.5 | 5.0 | 5.0 | 5.0 | 3.5 |
| PANTHENOL | 0.5 | 1.0 | 1.0 | 1.0 | 0.5 |
| EMULGADE ¹ | 0.2 | 0.2 | 0.2 | 0.2 | 0.2 |
| ISOHEXADECANE | 3.0 | 6.0 | 6.0 | 2.0 | - |
| ETHYLPARABEN | 0.15 | 0.15 | 0.15 | 0.15 | 0.15 |
| COCONUT OIL FRACTIONATED | ----- | ----- | | 4.0 | ----- |
| PROPYLPARABEN | 0.07 | 0.07 | 0.07 | 0.07 | 0.07 |
| STEARIC ACID | 0.1 | 0.1 | 0.1 | 0.1 | 0.1 |
| PEG-100 STEARATE ² | 0.1 | 0.1 | 0.1 | 0.1 | 0.1 |
| STEARYL ALCOHOL | 0.61 | 0.61 | 0.61 | 0.61 | 0.61 |
| CETYL ALCOHOL | 0.49 | 0.49 | 0.49 | 0.49 | 0.49 |
| BEHENYL ALCOHOL | 0.40 | 0.40 | 0.40 | 0.40 | 0.40 |
| ISOPROPYL ISOSTEARATE ³ | 1.5 | 3.0 | 1.5 | 1.5 | 0.65 |
| DL- α TOCOPHEROL ACETATE | 0.25 | 0.5 | 0.5 | 0.5 | 0.25 |
| MINERAL OIL | ----- | 1.0 | ----- | ----- | ----- |
| PETROLATUM | 2.0 | ----- | 1.5 | 1.5 | 0.5 |
| LUVIGEL EM ⁴ | 2.0 | 2.0 | ----- | 2.0 | 2.0 |
| SALCARE SC91 ⁵ | ----- | ----- | 2.0 | ----- | ----- |
| RHODASURF L⁷ | 0.0055 | 0.0055 | 0.0055 | 0.0055 | 0.0055 |
| VOLPO 3 ⁷ | 0.045 | 0.045 | 0.045 | 0.045 | 0.045 |
| SODIUM HYDROXIDE | 0.011 | 0.011 | 0.011 | 0.011 | ----- |
| NYLONPOLY WL 10 ⁸ | 1.0 | 1.0 | 1.0 | 1.0 | 1.0 |
| DRY FLO PLUS ⁹ | 1.0 | 1.5 | 2.0 | 2.0 | 1.0 |
| BENZYL ALCOHOL | 0.25 | 0.25 | 0.25 | 0.25 | 0.25 |
| DC 1503 ¹⁰ | 1.0 | 1.5 | 1.5 | 1.5 | 2.0 |
| PERFUME | 0.3 | 0.3 | 0.3 | 0.3 | 0.3 |
| OCTOCRYLENE | ----- | ----- | ----- | ----- | 1.0 |
| AVOBENZONE | ----- | ----- | ----- | ----- | 1.50 |
| OCTYL SALICYLATE | ----- | ----- | ----- | ----- | 3.5 |
| PHENYL BENZIMIDAZOLE SULPHONIC ACID | ----- | ----- | ----- | ----- | 1.0 |
| TRIETHANOLAMINE | ----- | ----- | ----- | ----- | 0.61 |

### EXAMPLES VI - IX

| **INGREDIENTS** | **VI** | **VII** | **VIII** | **IX** |
|---|---|---|---|---|
| | **% w/w** | **% w/w** | **% w/w** | **% w/w** |
| DEIONISED WATER | QS | QS | QS | QS |
| DISODIUM EDTA | 0.1 | 0.1 | 0.1 | 0.1 |
| GLYCERIN | 15.0 | 15.0 | 15.0 | 15.0 |
| NIACINAMIDE | 3.5 | 5.0 | 5.0 | 3.5 |
| PANTHENOL | 0.5 | 1.0 | 1.0 | 0.5 |
| EMULGADE ¹ | 0.2 | 0.2 | 0.2 | 0.2 |
| ISOHEXADECANE | 3.0 | 6.0 | 6.0 | 3.0 |
| ETHYLPARABEN | 0.15 | 0.15 | 0.15 | 0.15 |
| COCONUT OIL FRACTIONATED | -------- | -------- | -------- | -------- |
| PROPYLPARABEN | 0.07 | 0.07 | 0.07 | 0.07 |
| STEARIC ACID | 0.1 | 0.1 | 0.1 | 1.0 |
| PEG-100 STEARATE ² | 0.1 | 0.1 | 0.1 | 0.1 |
| STEARYL ALCOHOL | 0.61 | 0.61 | 0.61 | 0.79 |
| CETYL ALCOHOL | 0.49 | 0.49 | 0.49 | 0.64 |
| BEHENYL ALCOHOL | 0.40 | 0.40 | 0.40 | 0.52 |
| ISOPROPYL ISOSTEARATE ³ | 1.5 | 3.0 | 1.5 | 1.5 |
| DL- α TOCOPHEROL ACETATE | 0.25 | 0.5 | 0.5 | 0.25 |
| MINERAL OIL | -------- | 1.0 | -------- | -------- |
| PETROLATUM | 2.0 | -------- | 1.5 | 2.0 |
| LUVIGEL EM ⁴ | 2.0 | 2.0 | -------- | 0.5 |
| SALCARE SC91 ⁵ | -------- | -------- | 2.0 | -------- |
| RHODASURF L7 ⁶ | 0.0055 | 0.0055 | 0.0055 | 0.0055 |
| VOLPO 3 ⁷ | 0.045 | 0.045 | 0.045 | 0.045 |
| SODIUM HYDROXIDE | 0.011 | 0.011 | 0.011 | 0.011 |
| NYLONPOLY WL 10 ⁹ | 1.0 | 1.0 | 1.0 | 1.0 |
| DRY FLO PLUS ⁹ | 1.5 | 1.5 | 1.5 | 1.0 |
| BENZYL ALCOHOL | 0.25 | 0.25 | 0.25 | 0.25 |
| DC 1503 ¹⁰ | ----- | 1.5 | 1.5 | 1.0 |
| PERFUME | 0.3 | 0.3 | 0.3 | 0.3 |
| DC 9040 ¹¹ | 5.0 | ----- | ----- | ----- |
| TITANIUM DIOXIDE | ----- | 1.0 | 1.0 | ----- |
| IRON OXIDES | ----- | 0.11 | ----- | ----- |
| FD&C YELLOW 5 | ----- | ----- | 0.005 | ----- |
| FD&C RED 40 | ----- | ----- | 0.002 | ----- |

| | | | | |
|---|---|---|---|---|
| 1. Emulgade :Supplier: Cognis Deutchland GmbH, Paul-Thomas Strasse 56, D-40551 Dusseldorf, Germany. 2. PEG 100 Stearate supplied by ICI, PO Box 90, Wilton Centre, Middlesborough, Cleveland, England. TS6 8JE 3. Supplied by Scher Chemicals Inc, Industrial West, Clifton, NJ 07012 4. Luvigel EM:Supplier: BASF Plc, PO Box 4-Earl Road, Cheadle Hulme, Cheshire SK8 6QG 5. Salcare SC91: Supplier: CIBA Speciality Chemicals, Hulley Road, Macclesfield, Cheshire, SK10 2NX. 6. Rhodasurf :Supplier: Coldic, Staisty Close, Homewood Trading Estate, Chesterfield, Derbyshire S42 5UG 7. Volpo 3 : Supplier: Croda Oleochemicals, Cowick hall, Snaith Goole, East Yorkshire, Dn14 9AA 8. Nylonpoly: Supplier Optima Chemicals, Unit 17, Chiltern Business Village, Arundel Road, Uxbridge,Middlesex, UB8 2SN 9. Dry Flo :Supplier: National Starch Chemical Company, 10, Findeme Avenue, Bridgewater, NJ 08807, USA 10. & 11. DC 1503, DC9040: Supplied by Dow Coming, Kings Court, 185 Kinds Rd, Reading, Berks, RGI 4EX | | | | |

The compositions are made as follows:
A water phase is prepared by admixing all water soluble ingredients, except sodium hydroxide, in water and heating to about 80°C. A second premix is prepared by admixing of the oil soluble ingredients except the silicone oil (DC1503) and heating also to around 80°C. The oil phase is added to the water phase and sheared to form an emulsion.

The emulsion is cooled to 60°C and the polymeric thickener (Luvigel EM) and associated anionic surfactants (oleth 3, laureth 7) are then added. Sodium hydroxide solution is then added to neutralise to pH 6-7.5, except for examples where sunscreens are included. At 45-50°C the benzyl alcohol, DC1503, dyes and particles (including titanium dioxide and iron oxides) are added and the resulting product is sheared to ensure particle dispersion, de-agglomeration and homogeneity. The composition can then be cooled to 40°C and perfume can be added. The product can then be prepared for packaging.

Example IX can be packaged in a suitable can, pressurised with propane / butane / isobutene at 40psi and dispensed as a mousse.

## Claims

1. A cosmetic composition in the form of an oil-in-water emulsion, wherein the total level of oil phase components in the composition is between 5 and 15% by weight of the composition and wherein the continuous aqueous phase comprises from 0.1 to 2% cross-linked co-polymer by weight of the composition, the cross-linked co-polymer comprising;
i) from 35% to 94.5% water soluble anionic acrylic monomers by weight of the cross-linked co-polymer;
ii) from 5% to 65% water soluble non-ionic acrylate monomers by weight of the cross-linked co-polymer; and
iii) from 0.005% to 0.5% bifunctional monomeric crosslinking agent by weight of the cross-linked co-polymer;
the composition further comprising greater than 10% by weight of the composition of a water soluble skin benefit agent.

2. The cosmetic composition according to claim 1, wherein the cross-linked co- polymer comprises 50% to 70% water-soluble anionic monomers by weight of the cross-linked co-polymer.

3. The cosmetic composition according to claim 1 or claim 2 comprising from 0.2% to 0.8%, preferably from 0.3 to 0.7% by weight of the composition cross-linked copolymer.

4. The cosmetic composition according to claims 1 to 3, wherein the water soluble anionic acrylic monomer includes acrylic acid, methacrylic acid or mixtures thereof.

5. The cosmetic composition according to any of the preceding claims, wherein the water soluble non-ionic acrylate monomer includes acrylamide, methacrylamide, N-vinyl pyrolidone, water-soluble hydroxy-substituted acrylic or methacrylic esters or mixtures thereof.

6. The cosmetic composition according to any of the preceding claims, wherein the bifunctional monomeric crosslinking agent comprises methylene bis acrylamide, divinylpyrroline and allyl (meth) acrylate or mixtures thereof.

7. The cosmetic composition according to any of the preceding claims, wherein the water soluble skin benefit agent comprises vitamin B3 compounds, humectants, panthenol and derivatives, amino acids, vitamin C compounds or mixtures thereof.

8. The cosmetic composition according to claim 7, wherein the water-soluble skin benefit agent comprises a humectant, preferably a polyhydric alcohol.

9. The cosmetic composition according to claim 8, wherein the water-soluble benefit agent comprises glycerin.

10. The cosmetic composition according to any of the preceding claims, additionally comprising from 0.05% to 15% by weight of a surfactant.

11. The cosmetic composition according to claim 10, wherein the surfactant comprises a non-ionic surfactant.

12. The cosmetic composition according to claims 10 or 11, wherein the surfactant comprises a mixture of cetearyl glucosides and cetearyl alcohols.

13. The cosmetic composition according to any of the preceding claims additionally comprising from 0.1% to 10% by weight of an emollient.

14. The cosmetic composition according to any of the preceding claims additionally comprising from 0.1% to 5% by weight of a particulate material.

15. The cosmetic composition according to any of the preceding claims additionally comprising a silicone phase.

16. The cosmetic composition according to claim 15, wherein the silicone phase comprises a silicone gum / fluid blend.

## Patentansprüche

1. Kosmetische Zusammensetzung in Form einer Öl-in-Wasser-Emulsion, wobei der Gesamtanteil an Ölphasenkomponenten an der Zusammensetzung zwischen 5 und 15 Gew.-% der Zusammensetzung ausmacht, und wobei die kontinuierliche wässrige Phase, bezogen auf das Gewicht der Zusammensetzung, 0,1 bis 2 Gew.-% vernetztes Copolymer umfasst, wobei das vernetzte Copolymer umfas st;
i) bezogen auf das Gewicht des vernetzten Copolymers von 35 Gew.-% bis 94,5 Gew.-% wasserlösliche anionische Acrylmonomere;
ii) bezogen auf das Gewicht des vernetzten Copolymers von 5 Gew.-% bis 65 Gew.-% wasserunlösliche nichtionische Acrylatmonomere; und
iii) bezogen auf das Gewicht des vernetzten Copolymers 0,005 Gew.-% bis 0,5 Gew.-% bifunktionelles monomeres Vernetzungsmittel;
wobei die Zusammensetzung ferner zu mehr als 10 Gew.-% der Zusammensetzung einen wasserlöslichen, für die Haut vorteilhaften Wirkstoff umfasst.

2. Kosmetische Zusammensetzung nach Anspruch 1, wobei das vernetzte Co-polymer, bezogen auf das Gewicht des vernetzten Copolymers, 50 % bis 70 % wasserlösliche anionische Monomere umfasst.

3. Kosmetische Zusammensetzung nach Anspruch 1 oder Anspruch 2, die, bezogen auf das Gewicht der Zusammensetzung des vernetzten Copolymers, 0,2 % bis 0,8 %, vorzugsweise 0,3 bis 0,7 % umfasst.

4. Kosmetische Zusammensetzung nach Anspruch 1 bis 3, wobei das wasserlösliche anionische Acrylmonomer Acrylsäure, Methacrylsäure oder Mischungen davon aufweist.

5. Kosmetische Zusammensetzung nach einem der vorstehenden Ansprüche, wobei das wasserlösliche nichtionische Acrylatmonomer Acrylamid, Methacrylamid, N-Vinylpyrolidon, wasserlösliche hydroxysubstituierte Acryl- oder Methacrylester oder Mischungen davon aufweist.

6. Kosmetische Zusammensetzung nach einem der vorstehenden Ansprüche, wobei das bifunktionelle monomere Vernetzungsmittel Methylenbisacrylamid, Divinylpyrrolin und Allyl(meth)acrylat oder Mischungen davon umfasst.

7. Kosmetische Zusammensetzung nach einem der vorstehenden Ansprüche, wobei der wasserlösliche, für die Haut vorteilhafte Wirkstoff Vitamin B3-Verbindungen, Feuchthaltemittel, Panthenol und Derivate, Aminosäuren, Vitamin C-Verbindungen oder Mischungen davon umfasst.

8. Kosmetische Zusammensetzung nach Anspruch 7, wobei der wasserlösliche, für die Haut vorteilhafte Wirkstoff ein Feuchthaltemittel, vorzugsweise einen mehrwertigen Alkohol, umfasst.

9. Kosmetische Zusammensetzung nach Anspruch 8, wobei der wasserlösliche, für die Haut vorteilhafte Wirkstoff Glycerin umfasst.

10. Kosmetische Zusammensetzung nach einem der vorstehenden Ansprüche, außerdem umfassend von 0,05 Gew.-% bis 15 Gew.-% ein Tensid.

11. Kosmetische Zusammensetzung nach Anspruch 10, wobei das Tensid ein nicht-ionisches Tensid umfasst.

12. Kosmetische Zusammensetzung nach einem der Ansprüche 10 oder 11, wobei das Tensid eine Mischung aus Cetearylglucosiden und Cetearylalkoholen umfasst.

13. Kosmetische Zusammensetzung nach einem der vorstehenden Ansprüche, zusätzlich 0,1 Gew.-% bis 10 Gew.-% Weichmacher umfassend.

14. Kosmetische Zusammensetzung nach einem der vorstehenden Ansprüche, zusätzlich 0,1 Gew.-% bis 5 Gew.-% teilchenförmiges Material umfassend.

15. Kosmetische Zusammensetzung nach einem der vorstehenden Ansprüche, zusätzlich eine Silikonphase umfassend.

16. Kosmetische Zusammensetzung nach Anspruch 15, wobei die Silikonphase eine Silikongummi/Fluid-Mischung umfasst.

## Revendications

1. Composition cosmétique sous la forme d'une émulsion huile-dans-eau, dans laquelle le taux total de composants de la phase huileuse dans la composition est compris entre 5 et 15 % en poids de la composition et dans laquelle la phase aqueuse continue comprend de 0,1 à 2 % de copolymère réticulé en poids de la composition, le copolymère réticulé comprenant ;
i) de 35 % à 94,5 % de monomères acryliques anioniques hydrosolubles en poids du copolymère réticulé ;
ii) de 5 % à 65 % de monomères acrylate non ioniques hydrosolubles en poids du copolymère réticulé ; et
iii) de 0,005 % à 0,5 % d'agent de réticulation monomère bifonctionnel en poids du copolymère réticulé ;
la composition comprenant en outre plus de 10 % en poids de la composition d'un agent de soin cutané hydrosoluble.

2. Composition cosmétique selon la revendication 1, dans laquelle le copolymère réticulé comprend 50 % à 70 % de monomères anioniques hydrosolubles en poids du copolymère réticulé.

3. Composition cosmétique selon la revendication 1 ou la revendication 2, comprenant de 0,2 % à 0,8 %, de préférence de 0,3 à 0,7 % en poids de la composition de copolymère réticulé.

4. Composition cosmétique selon les revendications 1 à 3, dans laquelle le monomère acrylique anionique hydrosoluble inclut de l'acide acrylique, de l'acide méthacrylique ou leurs mélanges.

5. Composition cosmétique selon l'une quelconque des revendications précédentes, dans laquelle le monomère acrylate non ionique hydrosoluble inclut un acrylamide, un méthacrylamide, une N-vinyl pyrolidone, des esters acryliques ou méthacryliques à substitution hydroxy hydrosolubles ou leurs mélanges.

6. Composition cosmétique selon l'une quelconque des revendications précédentes, dans laquelle l'agent de réticulation monomère bifonctionnel comprend du méthylène bis acrylamide, de la divinylpyrroline et un (méth)acrylate d'allyle ou leurs mélanges.

7. Composition cosmétique selon l'une quelconque des revendications précédentes, dans laquelle l'agent de soin cutané hydrosoluble comprend des composés de vitamine B3, des humectants, du panthénol et dérivés, des acides aminés, des composés de vitamine C ou leurs mélanges.

8. Composition cosmétique selon la revendication 7, dans laquelle l'agent de soin cutané hydrosoluble comprend un humectant, de préférence un alcool polyhydrique.

9. Composition cosmétique selon la revendication 8, dans laquelle l'agent de soin hydrosoluble comprend de la glycérine.

10. Composition cosmétique selon l'une quelconque des revendications précédentes, comprenant en outre de 0,05 % à 15 % en poids d'un agent tensioactif.

11. Composition cosmétique selon la revendication 10, dans laquelle l'agent tensioactif comprend un agent tensioactif non ionique.

12. Composition cosmétique selon les revendications 10 ou 11, dans laquelle l'agent tensioactif comprend un mélange de cétéaryl glucosides et d'alcools cétéaryliques.

13. Composition cosmétique selon l'une quelconque des revendications précédentes, comprenant en outre de 0,1 % à 10 % en poids d'un émollient.

14. Composition cosmétique selon l'une quelconque des revendications précédentes, comprenant en outre de 0,1 % à 5 % en poids d'un matériau particulaire.

15. Composition cosmétique selon l'une quelconque des revendications précédentes, comprenant en outre une phase silicone.

16. Composition cosmétique selon la revendication 15, dans laquelle la phase silicone comprend un mélange gomme/fluide de silicone.
